# EUROPÄISCHE PATENTSCHRIFT

(11) **EP 1 242 687 B1**
(45) Veröffentlichungstag und Bekanntmachung des Hinweises auf die Patenterteilung: **06.08.2003**
(21) Anmeldenummer: 00985210.4
(22) Anmeldetag: 20.12.2000
(51) Int. Cl.: D21H 27/00, D21H 25/00, B31F 1/07

(54) **TISSUE- UND/ODER TISSUEÄHNLICHES MATERIAL SOWIE VERFAHREN ZU DESSEN HERSTELLUNG**
TISSUE AND/OR TISSUE-LIKE MATERIAL AND METHOD FOR THE PRODUCTION THEREOF
MATIERE PAPIER MENAGER ET/OU DE TYPE PAPIER MENAGER ET PROCEDE PERMETTANT DE LA PRODUIRE

(30) Priorität: 23.12.1999 DE 19962294
(43) Veröffentlichungstag der Anmeldung: 25.09.2002
(73) Patentinhaber: Metsä Tissue Oyj, 02100 ESPOO (FI)
(72) Erfinder: KLAPPERT, Ralf, 56587 Oberhonnefeld (DE)
(74) Vertreter: Patentanwälte Maxton & Langmaack
(86) Internationale Anmeldenummer: EP0013005
(87) Internationale Veröffentlichungsnummer: WO01048314

(56) Entgegenhaltungen:
- EP-A- 0 836 928
- US-A- 5 158 521
- US-A- 5 490 902
- US-A- 5 862 750

## Beschreibung

Die Erfindung betrifft ein Verfahren zur Herstellung von Tissue- und/oder tissueähnlichem Material für Toilettenpapiere, Taschentücher, Küchenrollen oder ähnlichem sowie ein mit diesem Verfahren hergestelltes Tissue- und/oder tissueähnliches Material.

Tissue- und/oder tissueähnliches Material findet vielfältig Verwendung. Aus derartigen Materialien werden beispielsweise Toilettenpapiere, Taschentücher, Küchenrollen, Servietten, Papiertischdecken, Saugeinlagen, Papierhandtücher, Wischtücher, Putztücher und vielerlei Produkte mehr hergestellt.

Im Sinne der Erfindung wird unter Tissue ein Erzeugnis verstanden, welches der DIN 6730 entspricht. Demnach ist Tissue ein Erzeugnis, welches ganz oder überwiegend aus Zellstoffasern besteht, mit einer feinen und weichen Kreppung und geschlossener Formation in der Papiermaschine mit einem Trockengehalt von mehr als 90% gekreppt wird, aus einer oder mehreren Lagen besteht, besonders saugfähig ist, eine flächenbezogene Masse der Einzellage von < 25 g/m² vor der Kreppung und eine Kreppnaßdehnung > 5% aufweist.

Im Sinne der Erfindung wird unter tissueähnlichem Material ein Material verstanden, welches im Unterschied zu Tissue-Material durch Veränderungen im Trockenvorgang hergestellt wird. Gemäß DIN 6730 ist tissueähnliches Material ein Erzeugnis überwiegend aus Zellstoffasern mit großer und weiter Kreppung und offener Formation, welches in der Papiermaschine trockengekreppt wird und aus einer oder mehreren Lagen besteht, wobei die flächenbezogene Masse einer Lage vor der Kreppung < 25 g/m² und die Naßkreppung einer Lage nach Kreppung > 25% ist. Das Material kann auch aus Altpapier hergestellt sein.

Für den Erfolg dieser Produkte bei den Endverbrauchern ist es insbesondere notwendig, deren Eigenschaften wie Festigkeit, Volumen, Griffigkeit, optische Eigenschaften, wie zum Beispiel Prägedesign oder Druckdesign, und insbesondere die Saugfähigkeit zu optimieren.

Um die gewünschten Eigenschaften der Endprodukte, wie Toilettenpapier oder ähnlichem, zu erreichen, ist es bekannt, die als Ausgangsmaterial verwendeten Tissue- und/oder tissueähnlichen Materialien mittels einer Kalanderstation zu behandeln. Hierdurch wird die Oberflächenstruktur des Ausgangsmaterials insbesondere im Hinblick auf die Weichheit, Glätte und das Volumen derselben beeinflußt. Bei diesem Verfahren werden die Ausgangsmaterialien zwischen mindestens zwei in aller Regel gegeneinanderlaufenden Walzen hindurchgeführt, wobei zwischen diesen beiden Walzen ein im Hinblick auf die gewünschte Eigenschaft der Endprodukte einstellbarer Spalt vorhanden ist. Insbesondere die Saugfähigkeit, vor allem eine Steigerung derselben, wird durch dieses Verfahren so gut wie überhaupt nicht beeinflußt.

Weiterhin ist es bekannt, zur Erreichung der gewünschten Eigenschaften keine Kalanderstation, sondern vielmehr eine Vorrichtung zu verwenden. Derartige Prägeverfahren werden überwiegend an bereits vordoublierten Material-Lagen verwendet, um die optischen und Volumen-Eigenschaften zu beeinflussen, sowie aus Lagenhaftungsgründen. Hier finden insbesondere zwei Verfahren Anwendung, nämlich die sogenannte Unions-Prägung und Stahl/Gummi-Prägung. Bei der Unions-Prägung wird das Ausgangsmaterial zwischen mindestens zwei gegeneinanderlaufende Walzen geführt, wobei diese Walzen auf der Oberfläche radial angeordnet Vorsprünge aufweisen, deren Querschnitt pyramidenstumpfförmig ist. Hierbei greifen die Vorsprünge der einen Walze in die durch die Vorsprünge der anderen Walze gebildeten Vertiefungen ein. Zwischen diesen beiden Walzen wird nun das Ausgangsmaterial hindurchgezogen, wobei es durch die Vorsprünge der beiden Walzen gleichmäßig geprägt wird. Die Prägung erfolgt hierbei entlang den Flanken der pyramidenstumpfförmigen Vorsprünge. Es entsteht ein Endprodukt, welches in regelmäßigen Abständen Prägepunkte aufweist. Auch durch dieses Verfahren wird jedoch insbesondere die Saugfähigkeit des verwendeten Materials so gut wie nicht oder auch zum Teil negativ beeinflußt.

Die Stahl/Gummi-Prägung verwendet ebenso wie die Unions-Prägung wenigstens zwei gegeneinanderlaufende Walzen, wobei jedoch nur die eine Walze pyramidenstumpfförmige Vorsprünge radial angeordnet auf der Oberfläche der einen Walze aufweist, wohingegen die andere Walze eine Walze mit einer elastischen Oberfläche aus beispielsweise Gummi ist. Die beiden gegeneinanderlaufenden Walzen sind hierbei derart eingestellt, daß die pyramidenstumpfförmigen Vorsprünge der einen Walze direkt in die elastische Schicht der weiteren Walze eintauchen und somit prägen. Wird nun eine Materialbahn des Ausgangsmaterials zwischen diesen beiden Walzen hindurchgezogen, so erfolgt eine Prägung durch die pyramidenstumpfförmigen Vorsprünge, die in die elastische Schicht hineintauchen. Auch durch dieses Verfahren werden die Materialeigenschaften Volumen, Griffigkeit, Oberflächenweichheit sowie die optischen Eigenschaften beeinflußt, jedoch so gut wie nicht oder auch negativ die Saugfähigkeit.

Wünschenswert wäre daher ein Verfahren zur Behandlung von Tissue- und/oder tissueähnlichen Materialien, durch welches sowohl die Materialeigenschaften Volumen, Griffigkeit, optische Eigenschaften wie beispielsweise Prägedesign oder Druckdesign beeinflußt werden als auch die Saugfähigkeit desselben.

US-A-5,490,902 offenbart ein Prägeverfahren zur Einreichung einer Zugfestigkeit von Papierprodukten nicht nur in longitudinaier, sondern auch in hierzu senkrechter Ausrichtung.

Hierzu wird die Papierbahn in eine Prägevorrichtung eingeführt, wobei die Prägemittel eine Anzahl von zwei unterschiedlich ausgebildeten Arten von Ausstülpungen aufweisen, die von dem Prägemittel abstehen, und wobei zumindest einige der Abschnitte der Prägemittel nahe der genannten Ausstülpungen so eingraviert bzw. vertieft sind, daß diese Abschnitte größere Höhen aufweisen als Abschnitte der genannten Ausstülpungen, die nicht den eingravierten bzw. vertieften Abschnitten nahe liegen. Dabei sind Ausstülpungen der einen Art in Maschinenrichtung, Ausstülpungen der anderen Art in Querrichtung hierzu angeordnet. Hierdurch wird eine Annäherung des Verhältnisses des Wertes der Längs-Zugfestigkeit an denjenigen der Quer-Zugfestigkeit erzielt.

US-A-5,158 521 offenbart einen Papierspender, bei welchem das enthaltene Papier bei Entnahme geprägt wird. Hierzu wird die Papierbahn zwischen zwei Walzen hindurchgeführt, wobei die eine Walze radial auf dieser angeordnete Rillen und die andere Walze vorstehende Prägepunkte aufweist, die in die Rillen der ersten Walze eingreifen. Hierdurch werden Schnitte in die Papierbahn eingebracht.

Der Erfindung liegt die Aufgabe zugrunde, ein Verfahren zur Herstellung von Tissue- und/oder tissueähnlichem Material zur Verfügung zu stellen, welches verbesserte optische und/oder haptische Eigenschaften und/oder eine erhöhte Saugeigenschaft auf weist.

Diese Aufgabe wird erfindungsgemäß gelöst durch ein Verfahren zur Herstellung von Tissue- und/oder tissueähnlichen Materialien, wobei
- in einem ersten Schritt in eine Vorrichtung, in welcher ein Anrißwerkzeug angeordnet ist, umfassend wenigstens zwei Anrißmittel,
   die Druckelemente mit Flanken aufweisen, die im wesentlichen parallel und/oder senkrecht zu einer durch den Mittelpunkt der Anrißmittel verlaufenden Symmetrieachse ausgerichtet sind, wobei die Anrißmittel so zueinander angeordnet sind, daß die Druckelemente des einen Anrißmittels in die durch zwei benachbarte Druckelemente des anderen Anrißmittels gebildeten Vertiefungen eingreifen,
   eine Materialbahn eingeführt wird,
- in einem zweiten Schritt die Materialbahn zwischen den Flanken gehalten und überdehnt wird, und
- in einem dritten Schritt das mit Mikroanrissen, welche auf einer Seite des Materials vorhanden sind, jedoch nicht durch dieses hindurchgehen, versehene Material der Vorrichtung entnommen wird.

Die Druckelemente im Sinne der Erfindung können sowohl höckerförmig als auch rillenförmig sein. Es ist auch möglich, daß das eine Anrißmittel höckerförmige Druckelemente aufweist, wohingegen das weitere Anrißmittel ein Netz aus zueinander senkrechten Rillen aufweist, in dessen Aussparungen die höckerförmigen Druckelemente des anderen Anrißmittels eingreifen.

Mittels des erfindungsgemäßen Verfahrens ist es möglich, Tissue- und/oder tissueähnliche Materialien zu erhalten, welche eine erhöhte Saugfähigkeit aufweisen und dabei den an diese Materialien gestellte Anforderungen im Hinblick auf die Weichheit und die Griffigkeit derselben entsprechen. Dadurch sind die mittels des erfindungsgemäßen Verfahrens hergestellten Tissue- und/oder tissueähnlichen Materialien insbesondere zur Verwendung als Toilettenpapiere, Taschentücher und Küchenrollen, Papiere mithin, von denen eine starke Saugfähigkeit gefordert wird, geeignet.

Durch die spezielle Ausgestaltung der Druckelemente erfolgt eine Bewegungsverhinderung der Bahn des Ausgangsmaterials, welche durch die wenigstens zwei Anrißmittel des Anrißwerkzeugs hindurchgeführt wird, wobei eine Überdehnung der Bahn über die Elastizitätsgrenze des Materials hinaus erfolgt. Die eingesetzte Materialbahn verändert dabei ihre Länge, was zu einer Überdehnung zuerst im elastischen Bereich und dann im plastischen Bereich des verwendeten Materials führt. Durch diese Überdehnung entstehen Mikroanrisse an der Materialbahn im Kopfbereich der Druckelemente. Ursache hierfür ist die durch die Ausgestaltung der Druckelemente ermöglichte genau definierte Klemmung der Materialbahn zwischen den Flanken der Druckelemente.

Die Größe der Mikroanrisse kann variiert werden durch eine genaue Abstimmung der Prägewalzen-Parameter, wie "Präge-Punkt-Höhe", "Präge-Punkt-Durchmesser", "Präge-Punkt-Form", sowie insbesondere dem Spalt zwischen den Druckelementen. Andererseits beeinflußt selbstverständlich auch das Ausgangsmaterial selbst die Größe der Mikroanrisse nach der Mikroanriß-Prägung. Zu nennen sind hier insbesondere die Dicke, die Dehnung und die Kreppung des eingesetzten Tissue- und/oder tissueähnlichen Materials.

Als Anrißwerkzeuge können Prägewerkzeuge mit zwei stempelförmigen, in einer Ebene ausgebildeten Anrißmitteln vorgesehen sein, auf denen die Druckelemente so auf den beiden Stempelflächen angeordnet sind, daß diese ineinandergreifen und die Materialbahn gehalten wird. Bevorzugt jedoch umfaßt das Anrißwerkzeug zwei Walzen als Anrißmittel, über welche die Mikroanrißprägung erfolgt. Auf diesen Walzen können die Druckelemente radial angeordnet sein.

Vorteilhafterweise werden durch das erfindungsgemäße Verfahren die Mikroanrisse quer zur Laufrichtung der Materialbahn gebildet. Hierdurch weist das mittels des erfindungsgemäßen Verfahrens hergestellte Tissue- und/oder tissueähnliche Material eine erhöhte Saugfähigkeit bei gleichwohl hoher Festigkeit auf. Bei Verarbeitung derartigen Tissue- und/oder tissueähnlichen Materials wird eine Bahn desselben durch eine Bearbeitungsstation gezogen. Werden nun die Mikroanrisse in Längsrichtung der Materialbahn gebildet, so besteht unter Umständen die Gefahr, daß diese durch den Zug der Materialbahn selbst in Längsrichtung vergrößert werden, was nachteilig zu Bahnrissen innerhalb der Maschine führen kann. Dies würde zu abrupten Maschinenstops infolge Bahnriß führen, wodurch Produktionsverluste entstehen würden. Zudem würde durch die dann entstehenden Vergrößerungen der Mikroanrisse die Saugfähigkeit nachteilig beeinflußt werden.

Vorteilhafterweise wird beim Eingreifen der Druckelemente in die Vertiefungen zwischen dem Grund dieser und dem Kopf des eingreifenden Druckelementes ein Spalt h₁ gebildet, der mindestens etwa der 1,5fachen Dicke der Materialbahn entspricht.

Bevorzugt wird beim Eingreifen der Druckelemente in die Vertiefungen zwischen dem Grund dieser und dem Kopf des eingreifenden Druckelementes des weiteren Anrißmittels der Spalt h₁ maximal etwa 1000µm, bevorzugt maximal etwa 300µm, weiter bevorzugt maximal etwa 250µm, betragen. Zwischen den Flanken benachbarter Druckelemente wird bevorzugt bei Eingreifen der Druckelemente der jeweiligen Walzen ineinander ein Spalt a von maximal 1000µm, bevorzugt von maximal etwa 300µm, weiter bevorzugt von etwa maximal 200µm, wobei Spaltabstände von etwa maximal 100µm bevorzugt sind, gebildet. Der Spalt muß so groß sein, daß die Materialbahn noch gehalten wird. Durch Variation des Spaltes a sowie des Spaltes h₁, insbesondere über die Ausbildung des Druckelements, ist es möglich, genau nach den an das fertige Produkte zu stellenden Anforderungen definierte Materialien zu erhalten, welche insbesondere über eine hohe Saugfähigkeit verfügen.

Die Erfindung betrifft weiterhin ein Tissue- und/oder tissueähnliches Material gemäß Anspruch 7, welches zumindest in Teilbereichen auf wenigstens einer seiner Oberflächen mit einer Vielzahl von Mikroanrissen versehen ist, die zur Oberfläche hin offen sind, und gemäß dem erfindungsgemäßen Verfahren hergestellt ist.

Das erfindungsgemäße Tissue- und/oder tissueähnliche Material weist eine hervorragende Qualität auf. Einerseits weist es insbesondere die gewünschten Eigenschaften im Hinblick auf die Griffigkeit und Weichheit desselben auf, welche im Hinblick auf die aus diesem herzustellenden Endprodukte von den Endverbrauchern gefordert wird, andererseits verfügt es über eine deutlich erhöhte Saugfähigkeit gegenüber Materialien, welche mit den im Stand der Technik bekannten Verfahren hergestellt wurden. So weist es beispielsweise im Vergleich mit Materialien, welche durch herkömmliche Prägeverfahren ("UNION" oder "Stahl/Gummi") hergestellt wurden, oder im Vergleich mit dem Ausgangsmaterial eine um mindestens etwa 25% gesteigerte Saugfähigkeit, ermittelt aus dem Absorptionsvermögen in Wasser gemäß der Norm CEN/TC 172/WG 8N 323, bei hervorragenden optischen und haptischen Eigenschaften, insbesondere der Weichheit und Griffigkeit, auf. Bevorzugt ist die Saugfähigkeit des erfindungsgemäßen Materials um mindestens etwa 40%, weiter bevorzugt um mehr als etwa 50%, gegenüber mit herkömmlichen Prägeverfahren hergestellten Materialien oder dem Ausgangsmaterial gesteigert.

Unter Mikroanrissen im Sinne der Erfindung werden Risse verstanden, welche auf einer Seite des erfindungsgemäßen Materials vorhanden sind, jedoch nicht durch dieses hindurchgehen. Hierdurch wird das Innere des Materials zu seiner Oberfläche hin geöffnet, so daß die Saugfähigkeit erhöht wird. Andererseits ist eine ausreichende Festigkeit gegeben, da die Risse nicht durch das Material hindurchgehen. Weiterhin weist das erfindungsgemäße Material ein besseres Aufnahmevermögen insbesondere für kleine Schmutzpartikel auf, da diese in den Mikroanrissen festgehalten werden. Hierdurch weist das erfindungsgemäße Material ein im Vergleich zu üblichen Materialien ohne Mikroanrisse deutlich erhöhtes Reinigungsvermögen auf.

Das erfindungsgemäße Material kann ein- oder mehrlagig, insbesondere zwei- bzw. dreilagig sein. Vorteilhaft an diesem ist insbesondere, daß es Materialkosteneinsparungen ermöglicht, insbesondere durch den Verzicht auf bei gleicher Saugfähigkeit notwendige Mehrmengen an Tissue- und/oder tissueähnlichen Ausgangsmaterialien. Das Material kann auch aus Altpapier hergestellt sein.

Das erfindungsgemäße Material weist also eine gezielte Zerstörung des Zellstoffasernverbundes und damit eine erhöhte Saugfähigkeit auf. Gleichwohl weist es eine für eine Anwendung ausreichende Festigkeit auf. Es vereint damit diese beiden offensichtlich im Widerspruch zueinander stehenden Eigenschaften.

Insbesondere weisen die in dem Tissue- und/oder tissueähnlichen Material enthaltenen Mikroanrisse eine Größe auf, die einerseits ausreicht, um Flüssigkeit aufzunehmen, andererseits noch so klein ist, daß die Flüssigkeit nicht aus diesen entweichen kann.

Es ist insbesondere vorteilhaft, daß die Mikroanrisse im Material quer zur Faserrichtung des Materialbandes ausgerichtet sind. Hierdurch wird erreicht, daß trotz der Mikroanrisse das Festigkeitsverhalten des Materials sehr gut ist. Bevorzugt sind die Mikroanrisse in dem erfindungsgemäßen Material in Teilbereichen auf der Oberfläche des Materials angeordnet. Weiter bevorzugt sind die Mikroanrisse in Teilbereichen abwechselnd auf der Ober- und der Unterseite des Materials angeordnet. Die Teilbereiche können sich teilweise untereinander berühren und eine unterschiedliche Größe aufweisen. Vorteilhafterweise sind sie jedoch voneinander getrennt, insbesondere durch Bereiche, die unbehandeltes und/oder lediglich leicht überdehntes Material aufweisen. Derartiges Material weist trotz der gezielten Zerstörung des Faserverbundes ausgezeichnete Festigkeiten bei gleichwohl hoher Saugfähigkeit und weichem textilem Griff auf.

Schließlich betrifft die Erfindung auch die Verwendung eines Tissue- und/oder tissueähnlichen Materials mit Mikroanrissen als Toilettenpapier, Taschentücher, Küchenrollen, Servietten, Papiertischdecken, Saugeinlagen, Papierhandtücher, Wischtücher und/oder Putztücher.

Die vorstehenden und weitere Vorteile der Erfindung werden anhand der nachfolgenden schematischen Zeichnungen erläutert. Es zeigen:
- Fig. 1: eine Seitenansicht einer Vorrichtung zur Herstellung des erfindungsgemäßen Materials;
- Fig. 2: Einzelheit A der Fig. 1;
- Fig. 3: Einzelheit B der Fig. 2;
- Fig. 4: um 90° gedrehte Ansicht der Einzelheit B; und
- Fig. 5: skizzenhafte Darstellung der Verteilung der Mikroanrisse auf dem erfindungsgemäßen Material.

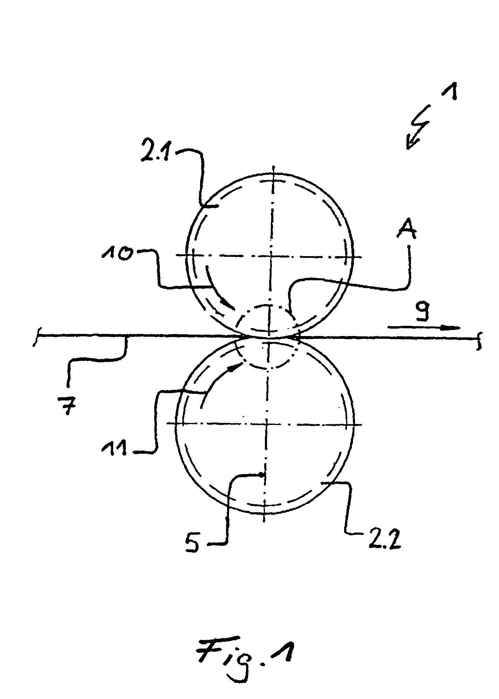

Fig. 1 zeigt eine insgesamt mit dem Bezugszeichen 1 versehene Vorrichtung mit einem Anrißwerkzeug, bestehend aus zwei Walzen 2.1 und 2.2 als Anrißmittel, zwischen denen eine Materialbahn 7 in Laufrichtung des Pfeiles 9 durch diese Walzen 2.1 und 2.2 hindurchgezogen wird. Die beiden Walzen 2.1 und 2.2 laufen gegeneinander, wie dies durch die Pfeile 10 und 11 angedeutet ist.

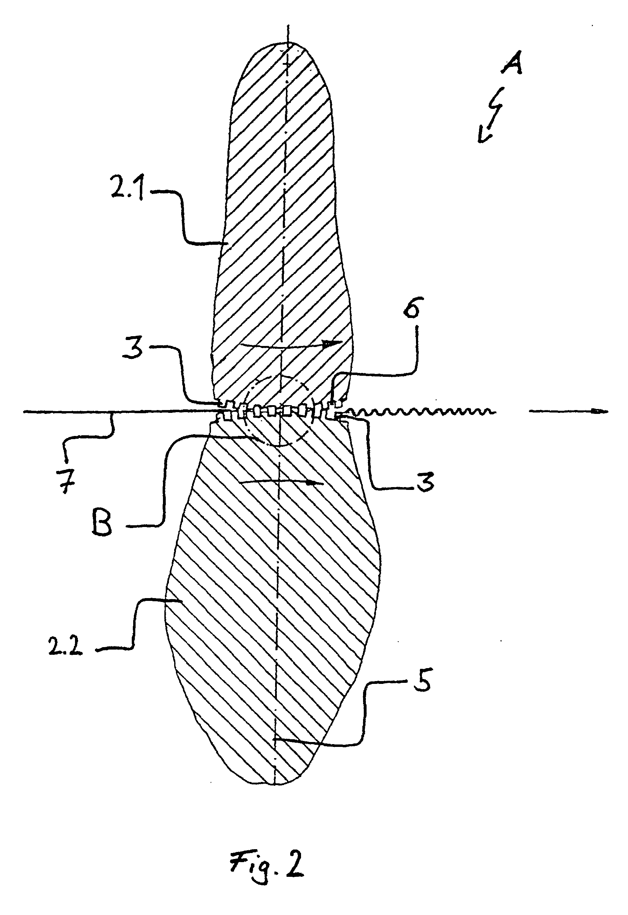

Die Einzelheit A der Fig. 1 ist in Fig. 2 perspektivisch dargestellt. Die beiden Walzen 2.1 und 2.2 weisen auf ihrer Oberfläche eine Vielzahl von Druckelementen 3.1, 3.2 auf, deren Flanken in etwa parallel und senkrecht dazu zu einer durch den Mittelpunkt der Walzen 2.1 und 2.2 verlaufenden Symmetrieachse 5 ausgerichtet sind. Hierbei greift ein Druckelement 3.1 einer Walze 2.1 in die durch zwei radial benachbarte Druckelemente 3.2 der anderen Walze 2.2 gebildeten Vertiefungen 6.1 ein.

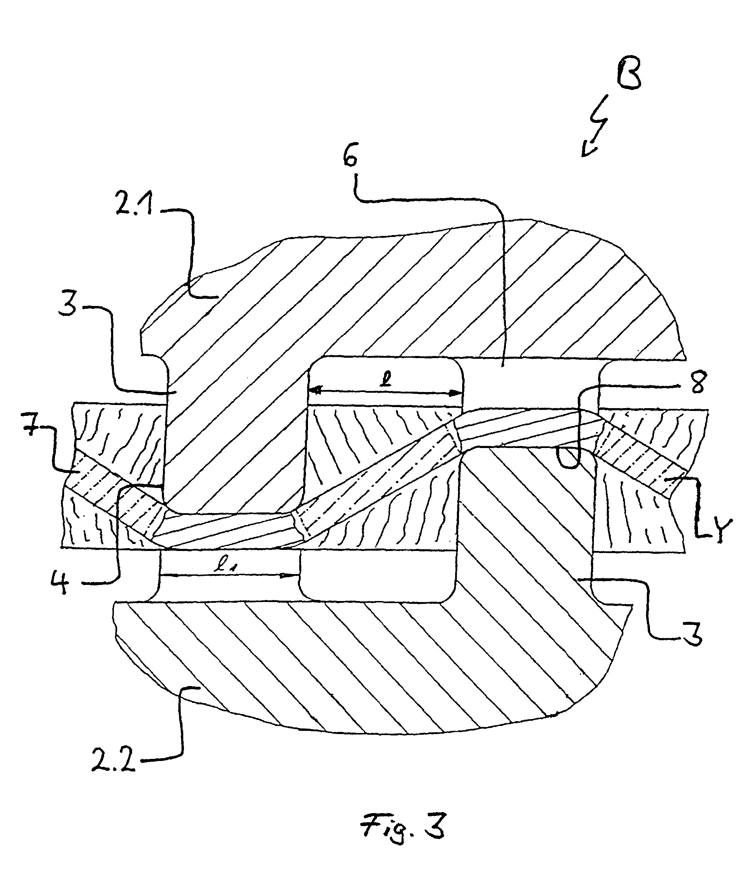

Fig. 3 zeigt eine Einzelheit B der Fig. 2. Die durch die beiden gegeneinanderlaufenden Walzen 2.1 und 2.2 hindurchgeführte Materialbahn 7 wird durch die Druckelemente 3.1, 3.2 gehalten und gedehnt. Diese Druckelemente weisen eine Länge l₁ auf, welche üblicherweise in einem Bereich von etwa 400 bis 2000µm liegt, oder aber im Fall einer Rillenwalze endlos ist. Der Spalt 1 zwischen zwei radial benachbarten Druckelementen 3 liegt vorzugsweise in einem Bereich bis etwa 2500µm. Im Falle der Verwendung einer Rillenwalze ist dieser radiale Spalt 1 nicht vorhanden.

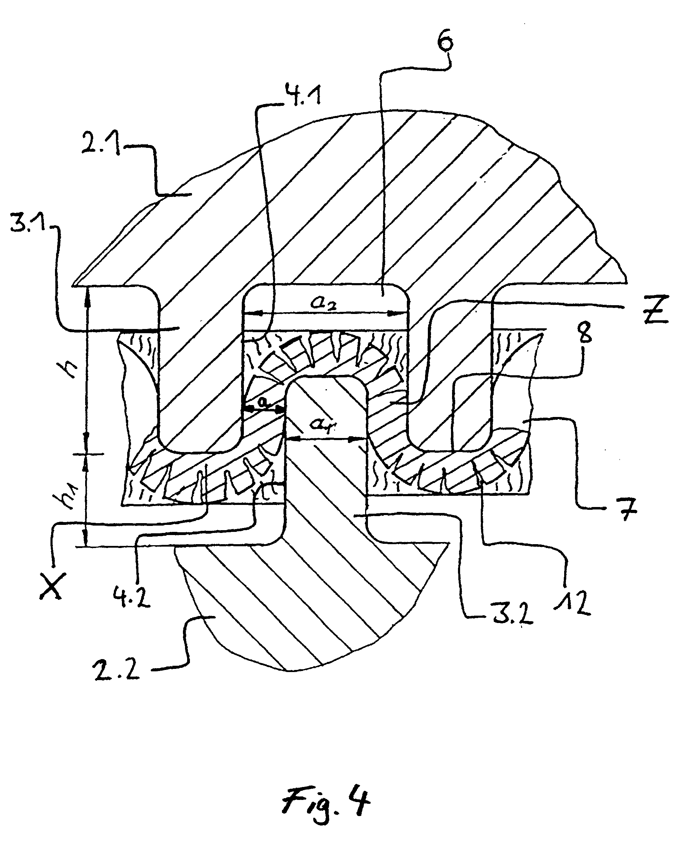

Fig. 4 zeigt eine um 90° gedrehte Seitenansicht der Fig. 3. Die durch die beiden Walzen 2.1 und 2.2 geführte Materialbahn 7 wird zwischen den Flanken 4.1 und 4.2 der benachbarten Druckelemente 3.1 und 3.2 gehalten. Aufgrund dieser Bewegungsverhinderung der Materialbahn 7 senkrecht zur Laufrichtung entstehen Mikroanrisse 12 im Bereich des Kopfes 8 der Druckelemente 3.1, 3.2. Der Kopf 8 der Druckelemente 3 kann dabei vielfältig ausgestaltet sein, beispielsweise als Oval, Kreis, Rechteck, Raute oder in anderen Formen. Es kann sowohl eben als auch leicht gewölbt sein. Es ist jedoch auch möglich, die Druckelemente 3.1, 3.2 als radial oder axial um die Walze laufende Rillen auszubilden (Rillenwalze). Es erfolgt also eine Überdehnung über den Umfang der Prägepunkte. Die Druckelemente 3.1, 3.2 weisen eine axiale Breite a₁ auf, welche in einem Bereich von etwa 200 bis 1000µm liegt, aber auch unendlich sein kann (Rillenwalze). Aus der axialen Breite a₁ der Druckelemente 3.1, 3.2 und der axialen Breite a₂ der Vertiefung 6.2 läßt sich der axiale Spalt a ermitteln. Dieser liegt in einem Bereich bis maximal 1000µm, bevorzugt in einem Bereich bis maximal etwa 200µm, bevorzugt ist der axiale Spalt a kleiner gleich 100µm. Die Druckelemente 3.1, 3.2 weisen eine Höhe h in einem Bereich von etwa 300 bis 1500µm auf. Der Spalt h₁ zwischen dem Kopf 8 der Druckelemente 3 und dem Grund der Vertiefung 6.2 liegt in einem Bereich von etwa 200 bis 1000µm und beträgt mindestens etwa der 1,5fachen Dicke der eingezogenen Materialbahn 7.

Im Bereich Y der Fig. 3 erfolgt eine leichte Überdehnung der Materialbahn 7, wodurch jedoch keine Mikroanrisse 12 erzeugt werden. Im Bereich X der Fig. 4 entstehen Mikroanrisse 12, der Bereich Z bezeichnet den Haltebereich zwischen den Flanken 4.1 und 4.2 zweier benachbarter Druckelemente 3.1 und 3.2 in axialer Richtung.

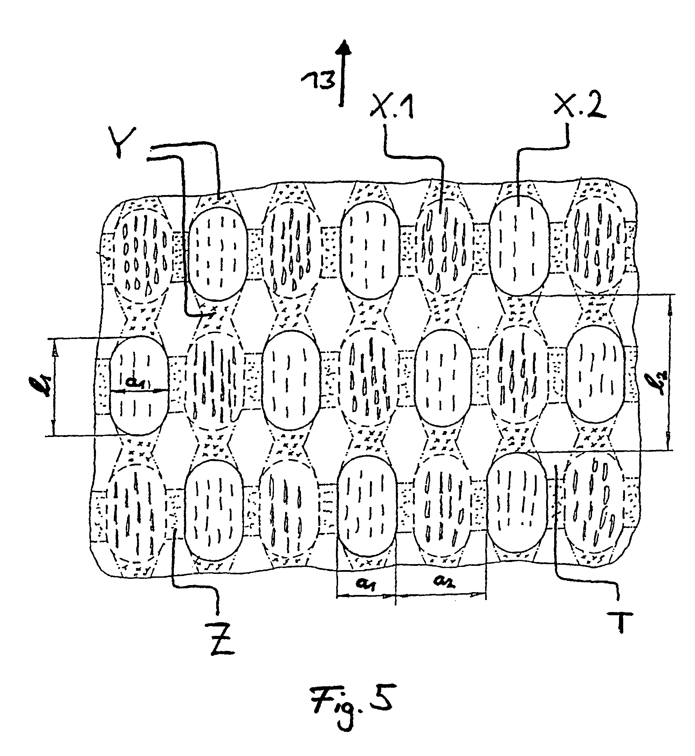

Fig. 5 zeigt eine skizzenhafte Darstellung der verschiedenen Bereiche T, X, Y und Z des erfindungsgemäßen Materials, wobei dieses sowohl mit Walzen als Anrißmitteln der Vorrichtung 1 als auch unter Verwendung von Anrißwerkzeugen mit stempelförmigen Anrißmitteln hergestellt sein kann. Die Bereiche X sind sowohl auf der Oberseite X.1 als auch auf der Unterseite X.2 der Materialbahn 7 nach Durchlaufen der Vorrichtung 1 abwechselnd angeordnet und weisen Mikroanrisse 12 in Laufrichtung der Materialbahn 7 auf. An die Bereiche X.1 und X.2 angrenzende Zonen in Laufrichtung 13 der Materialbahn 7 - Bereiche Y - zeigen leicht überdehntes Ausgangsmaterial 7, jedoch keine Mikroanrisse 12. Den Bereichen X.1 und X.2 benachbarte Zonen zeigen den Haltebereich der Materialbahn 7 - Bereiche Z - , quer zur Laufrichtung 13 durch die Flanken 4.1 und 4.2 der Druckelemente 3.1 und 3.2 erzeugt, ohne Mikroanrisse 12. In diesem Bereich treten Quetsch- bzw. Klemmungseffekte auf. Die zwischen den Bereichen X, Y und Z angeordneten Bereiche T sind nach Durchlaufen der Vorrichtung 1 unbehandelt. Durch einfache Vertauschung bzw. Änderung der Druckelemente 3 auf den Walzen 2 und der Geometrie und Abmessungen der Spalte a und 1 lassen sich Mikroanrisse 12 quer oder sowohl senkrecht als auch quer in gleichen oder angrenzenden Teilbereichen zur Laufrichtung erzeugen. Dies gilt insbesondere auch im Falle der Verwendung von Anrißwerkzeugen mit stempelförmigen Anrißmitteln anstatt von Walzen (2.1, 2.2).

Im Falle der Verwendung einer Rillenwalze (axial und/oder radial) mit radialen Rillen würden keine Bereiche Y erzeugt. In dem Fall, in dem Mikroanrisse sowohl quer als auch senkrecht durch entsprechende Anordnung der Druckelemente erzeugt werden, weisen gegebenenfalls auch die Bereiche T Mikroanrisse auf, und zwar beispielsweise quer zu denjenigen in den Bereichen X.1 und X.2, die Bereiche Y hingegen gehen zumindest teilweise in Bereiche Z über. Im Falle von ausschließlich quer zur Laufrichtung erzeugten Mikroanrissen sind die Bereiche Z und Y vertauscht. Es ist auch möglich, eine Materialbahn 7 mehr als einmal durch eine Vorrichtung 1 laufen zu lassen. Hierdurch erfolgt eine gleichmäßige Verteilung der Mikroanrisse, jedoch ist das mehrmalige Durchlaufen auf wenige Läufe in Abhängigkeit von der verwendeten Materialbahn begrenzt, da ansonsten die Festigkeit des behandelten Materials herabgesetzt würde. Bevorzugt werden die Mikroanrisse 12 quer zur Faserrichtung der eingezogenen Materialbahn 7 erzeugt.

Mittels der vorstehend definierten Vorrichtung wurde ein Tissuematerial verarbeitet mit einer Formatur im Bereich von 18,5 bis 22 g/m². Die Dicke des eingesetzten Tissue-Materials betrug etwa 150µm. Die Länge l₁ der Druckelemente 3.1, 3.2 betrug 700µm, die Breite a₁ der Druckelemente 3.1, 3.2 350µm. Die Höhe der Druckelemente 3.1, 3.2 betrug 500µm, die Länge l₂ der Vertiefung 6.1 1700µm und die Breite a₂ der Vertiefung 6.2 800µm. Somit betrug der Spalt a 225µm, der Spalt 1 500µm und der Spalt h₁ 250µm. Hiermit wurde ein erfindungsgemäßes Tissuematerial hergestellt, welches im Unterschied zum unbehandelten Material eine um 25% gesteigerte Saugfähigkeit und einen weichen textilen Griff aufwies. Durch die Reduzierung des Spaltes a wird eine Steigerung der Klemmwirkung und eine gesteigerte Mikroanrißbildung erzielt, was zu einer 50%igen Steigerung der Saugfähigkeit führt.

Mittels der Vorrichtung 1 ist es auch möglich, nicht nur einlagiges Material, wie vorstehend beschrieben, zu verarbeiten, sondern auch bereits doublierte Tissue- und/oder tissueähnliche Materialien in einer Vorstufe der Fertigverarbeitung zur Oberflächenbehandlung zu verwenden, um nicht nur die Saugfähigkeit dieses Materials zu erhöhen, sondern auch den textilen Charakter, insbesondere die Weichheit und Griffigkeit der Oberfläche des Materials, zu verändern.

## Patentansprüche

1. Verfahren zur Herstellung von Tissue- und/oder tissueähnlichem Material, wobei
- in einem ersten Schritt in eine Vorrichtung (1), in welcher ein Anrißwerkzeug angeordnet ist, umfassend wenigstens zwei Anrißmittel (2.1, 2.2),
die Druckelemente (3) mit Flanken (4) aufweisen, die im wesentlichen parallel und/oder senkrecht zu einer durch den Mittelpunkt der Anrißmittel (2.1, 2.2) verlaufenden Symmetrieachse (5) ausgerichtet sind, wobei die Anrißmittel (2.1, 2.2) so zueinander angeordnet sind, daß die Druckelemente (3.1) des einen Anrißmittels (2.1) in die durch zwei benachbarte Druckelemente (3.2) des anderen Anrißmittels (2.2) gebildeten Vertiefungen (6) eingreifen,
eine Materialbahn (7) eingeführt wird,
- in einem zweiten Schritt die Materialbahn (7) zwischen den Flanken (4.1, 4.2) gehalten und überdehnt wird, und
- in einem dritten Schritt das mit Mikroanrissen, welche auf einer Seite des Materials vorhanden sind, jedoch nicht durch dieses hindurchgehen, versehene Material der Vorrichtung (1) entnommen wird.

2. Verfahren nach Anspruch 1, **dadurch gekennzeichnet, daß** das Anrißwerkzeug als Anrißmittel zwei Walzen (2.1, 2.2) umfaßt.

3. Verfahren nach einem der Ansprüche 1 oder 2, **dadurch gekennzeichnet, daß** die Mikroanrisse quer zur Laufrichtung der Materialbahn (7) im Material gebildet werden.

4. Verfahren nach einem der vorhergehenden Ansprüche, **dadurch gekennzeichnet, daß** die Walzen (2.1, 2.2) der Vorrichtung (1) gegeneinander laufen.

5. Verfahren nach einem der vorhergehenden Ansprüche, **dadurch gekennzeichnet, daß** beim Eingreifen der Druckelemente (3.1, 3.2) in die Vertiefungen (6) zwischen dem Grund dieser und dem Kopf (8) des eingreifenden Druckelementes (3) ein Spalt h₁ gebildet wird, der mindestens etwa der 1,5fachen Dicke der Materialbahn (7) entspricht.

6. Verfahren nach einem der vorhergehenden Ansprüche, **dadurch gekennzeichnet, daß** bei Eingreifen der Druckelemente (3) ineinander zwischen den Flanken (4.1, 4.2) benachbarter Druckelemente (3.1, 3.2) ein Spalt a von maximal etwa 1000µm gebildet wird.

7. Tissue- und/oder tissueähnliches Material für Toilettenpapiere, Taschentücher, Küchenrollen oder ähnliches, erhältlich durch das Verfahren gemäß einem der Ansprüche 1 bis 6, welches zumindest in Teilbereichen auf wenigstens einer seiner Oberflächen mit einer Vielzahl von Mikroanrissen (12) versehen ist, die zur Oberfläche hin offen sind, jedoch nicht durch das Material hindurchgehen.

8. Tissue- und/oder tissueähnliches Material gemäß Anspruch 7, **dadurch gekennzeichnet, daß** die Mikroanrisse (12) im Material quer zur Faserrichtung des Materials ausgerichtet sind.

9. Tissue- und/oder tissueähnliches Material gemäß einem der Ansprüche 7 oder 8, **dadurch gekennzeichnet, daß** die Mikroanrisse (12) in Teilbereichen auf der Oberfläche angeordnet sind.

10. Tissue- und/oder tissueähnliches Material gemäß einem der Ansprüche 7 bis 9, **dadurch gekennzeichnet, daß** die Mikroanrisse (12) in Teilbereichen abwechselnd auf der Ober- und der Unterseite des Materials angeordnet sind.

11. Verwendung eines Tissue- und/oder tissueähnlichen Materials mit Mikrorissen 12 gemäß den vorhergehenden Ansprüchen als Toilettenpapier, Taschentücher, Küchenrollen, Servietten, Papiertischdecken, Saugeinlagen, Papierhandtücher, Wischtücher und/oder Putztücher.

## Claims

1. Method for the production of tissue and/or tissue-like material, whereby
- as a first step, into a device (1) comprising a roughing tool comprising at least two roughing units (2.1, 2.2),
which comprise pressure elements (3) with flanks (4), which are aligned substantially parallel and/or vertical to an axis of symmetry (5) running through the centre of said roughing units (2.1,2.2), whereby said roughing units (2.1, 2.2) are arranged to one another in a way that said pressure elements (3.1) of one said roughing unit (2.1) grip into the grooves (6) formed by two of said neighbouring pressure elements (3.2) of said other roughing unit (2.2),
a web of material (7) is inserted,
- as a second step, said web of material (7) is gripped between said flanks (4.1, 4.2) and overstretched, and
- as a third step, said material, provided with micro-fissures, which exist on one side of said material, but do not go through it, is removed from said device (1).

2. Method according to Claim 1, **characterised in that** said roughing tool comprises as said roughing units two rollers (2.1, 2.2).

3. Method according to any of the Claims 1 or 2, **characterised in that** said micro-fissures are formed in said material across the machine direction of said web of material (7).

4. Method according to any of the preceding Claims, **characterised in that** said rollers (2.1, 2.2) of said device (1) rotate in opposite direction to one another.

5. Method according to any of the preceding Claims, **characterised in that** on gripping of said pressure elements (3.1, 3.2) into said grooves (6) between the base of such a said groove and the head (8) of said gripping pressure element (3) a gap h₁ is being formed which equals at least about 1.5 times the thickness of said web of material (7).

6. Method according to any of the preceding Claims, **characterised in that** on gripping of said pressure elements (3) into one another a gap "a" of at most about 1000µm is being formed between said flanks (4.1, 4.2) of said corresponding pressure elements (3.1, 3.2).

7. Tissue and/or tissue-like material for toilet paper, handkerchiefs, kitchen rolls and the like, obtainable through said method according to one of the Claims 1 to 6, which at least in partial areas on at least one of its surfaces is provided with a multitude of micro-fissures (12), which are open toward the surface, but do not go through said material.

8. Tissue and/or tissue-like material according to Claim 7, **characterised in that** said micro-fissures (12) are aligned in said material across the grain of said material.

9. Tissue and/or tissue-like material according to Claim 7 or 8, **characterised in that** said micro-fissures (12) are arranged in partial areas on the surface.

10. Tissue and/or tissue-like material according to any of the Claims 7 to 9, **characterised in that** said micro-fissures (12) are arranged in partial areas on the top and bottom side alternately.

11. Use of a tissue and/or tissue-like material with micro-fissures (12) according to the preceding Claims as toilet paper, handkerchiefs, kitchen rolls, napkins, paper table-cloths, absorbing layers, paper towels, cleaning cloths and/or floor cloths.

## Revendications

1. Procédé de fabrication d'un matériau en tissu ouaté et/ou semblable à du tissu ouaté, dans lequel :
- lors d'une première étape, une bande de matériau (7) est insérée dans un dispositif (1), dans lequel est agencé un outil de déchirure, comprenant au moins deux moyens de déchirure (2.1, 2.2),
qui présentent des éléments de pression (3) avec des flancs (4) qui sont orientés de manière essentiellement parallèle et/ou perpendiculaire à un axe de symétrie (5) passant par le point milieu des moyens de déchirure (2.1, 2.2), les moyens de déchirure (2.1, 2.2) étant agencés l'un par rapport à l'autre de telle sorte que les éléments de pression (3.1) de l'un des moyens de déchirure (2.1) engrènent dans les évidements (6) formés par deux éléments de pression (3.2) adjacents de l'autre moyen de déchirure (2.2),
- lors d'une deuxième étape, la bande de matériau (7) est maintenue entre les flancs (4.1, 4.2) et étirée, et
- lors d'une troisième étape, le matériau muni de microdéchirures, présentes sur un côté du matériau, mais ne traversant pas celui-ci, est retiré du dispositif (1).

2. Procédé selon la revendication 1, **caractérisé en ce que** l'outil de déchirure comporte, en guise de moyens de déchirure, deux rouleaux (2.1, 2.2).

3. Procédé selon l'une quelconque des revendications 1 ou 2, **caractérisé en ce que** les microdéchirures sont formées dans le matériau transversalement au sens de défilement de la bande de matériau (7).

4. Procédé selon l'une quelconque des revendications précédentes, **caractérisé en ce que** les rouleaux (2.1, 2.2) du dispositif (1) roulent l'un contre l'autre.

5. Procédé selon l'une quelconque des revendications précédentes, **caractérisé en ce que** lors de l'engrènement des éléments de pression (3.1, 3.2) dans les évidements (6), il se forme entre le fond de ceux-ci et la tête (8) de l'élément de pression (3) engrenant un entrefer h₁ qui correspond à au moins environ 1 fois et demie l'épaisseur de la bande de matériau (7).

6. Procédé selon l'une quelconque des revendications précédentes, **caractérisé en ce que** lors de l'engrènement des éléments de pression (3) l'un dans l'autre, il se forme entre les flancs (4.1, 4.2) d'éléments de pression adjacents (31, 3.2), un entrefer d'environ 1 000 µm au maximum.

7. Matériau en tissu ouaté et/ou semblable à du tissu ouaté pour papier toilette, mouchoirs de poche, essuie-tout ou similaire, pouvant être obtenu par le procédé selon l'une quelconque des revendications 1 à 6, lequel est muni, au moins dans des certaines zones sur au moins une de ses surfaces, d'une pluralité de microdéchirures (12), qui sont ouvertes vers la surface, mais qui ne traversent cependant pas le matériau.

8. Matériau en tissu ouaté et/ou semblable à du tissu ouaté selon la revendication 7, **caractérisé en ce que** les microdéchirures (12) dans le matériau sont orientées transversalement au sens des fibres du matériau.

9. Matériau en tissu ouaté et/ou semblable à du tissu ouaté selon la revendication 7 ou 8, **caractérisé en ce que** les microdéchirures (12) sont agencées dans certaines zones de la surface.

10. Matériau en tissu ouaté et/ou semblable à du tissu ouaté selon l'une quelconque des revendications 7 à 9, **caractérisé en ce que** les microdéchirures (12) sont agencées dans certaines zones en alternance sur le dessus et le dessous du matériau.

11. Utilisation d'un matériau en tissu ouaté et/ou semblable à du tissu ouaté avec microdéchirures (12) selon les revendications précédentes comme papier toilette, mouchoirs de poche, essuie-tout, serviettes, nappes de table en papier, couches d'absorption, mouchoirs en papier, feutres et/ou torchons.
